# EUROPEAN PATENT APPLICATION

(11) **EP 2 243 426 A1**
(43) Date of publication of application: **27.10.2010**
(21) Application number: 08862071.1
(22) Date of filing: 19.12.2008
(51) Int. Cl.: A61B 5/151, A61B 5/157

(54) **LANCET DEVICE AND PUNCTURE DEVICE**

(30) Priority: 19.12.2007 JP 2007328086
(71) Applicant: ARKRAY, Inc., Minami-ku Kyoto-shi Kyoto 601-8045 (JP)
(72) Inventor: KIMURA, Sadaaki, Kyoto-shi Kyoto 601-8045 (JP)
(74) Representative: HOFFMANN EITLE
(86) International application number: PCT/JP2008/073263
(87) International publication number: WO 2009/078488

(57) **Abstract**

The present invention relates to a lancet device 2 which is attached to a puncture device and is used for piercing a target site, and which retains a lancet 4 having a puncture needle in an internal space 35 of a casing 3. The casing 3 has a visible region 36 through which a leading end of the lancet 4 is visually recognizable. The casing 3 comprises, for example, a main body 30 which has the internal space 35, and a cover 31 which covers a part of the internal space35. The visible region 36 has an opened part in the internals space 35 which is not covered by the cover 31 and is adjacent to the cover 31.

## Description

### Technical Field

The present invention relates to a lancet device and a puncture device used when, for example, a specimen, such as a blood or an interstitial fluid, is extracted through a skin.

### Background Art

When a specific constituent in a specimen is analyzed using the specimen like a blood extracted through a skin, it is necessary to pierce the skin, and to supply the specimen caused to flow out through the skin to an analytical instrument like a biosensor. The skin is pierced using a puncture device to which, for example, a lancet is attached. Meanwhile, the blood is supplied to the analytical instrument by, for example, contacting a specimen inlet of the analytical instrument with a blood flow-out site of the skin. The blood is supplied to the analytical instrument before the analytical instrument is attached to a blood analyzer, or with the analytical instrument being attached to the blood analyzer.

According to such a technique, it is necessary to separately carry the puncture device and the blood analyzer, and to change the devices when in use, which results in a poor usability. Moreover, it is necessary to attach the lancet to the puncture device when the skin is pierced, and to attach the analytical instrument to the analyzer when the blood is analyzed, which forces a user to carry out a bothersome operation. In particular, for a person with a weakened eyesight, attachment of the lancet in the puncture device and of the biosensor to the analyzer are bothersome, and appropriately contacting the analytical instrument with the blood flow-out site of the skin is also a difficult work.

On the other hand, there is a puncture device which can be united with a blood analyzer (see, for example, Patent Literatures 1 and 2). In this case, the blood analyzer and the puncture device can be used in a united condition, or can be used individually, which results in improvement of the usability. However, attachment of a lancet or an analytical instrument and contacting of the analytical instrument with a blood flow-out site appropriately are not still improved, which leaves botheration and difficulty.

There is proposed a device having a lancet and an analytical instrument integrated together so as to improve the operability and the workability (see, for example, Patent Literature 3). Such device can pierce a skin with a lancet and can supply a specimen to an analytical instrument by merely being attached to an analyzer and being used as it is.

However, according to the integrated-type device, in general, as the lancet is retained in a casing, it is difficult for a user to check the lancet or a flow-out condition of a blood through a skin when the skin is pierced. As a result, insufficient supply of a specimen to the analytical instrument or measurement error is likely to happen originating from the user's anxiety when the skin is pierced, or difficulty of checking of a blood flow-out condition through the skin.

Patent Literature 1: Japan Patent No. 3569228
Patent Literature 2: Japan National Patent Publication No. 2001-524680
Patent Literature 3: Unexamined Japanese Patent Application KOKAI Publication No. 2004-33376

### Disclosure of Invention

### Problem to be solved by the Invention

It is an object of the present invention to suppress any insufficient supply of a specimen to an analytical instrument or any occurrence of measurement error, while at the same time, to eliminate the anxiety of a user as much as possible when a skin is pierced.

### Means for solving the problem

The present invention according to a first aspect provides a lancet device which is attached to a puncture device and is used for piercing a target site, and which retains a lancet comprising a puncture needle in an internal space of a casing, wherein the casing includes a visible region through which a leading end of the lancet is visually recognizable.

The casing comprises, for example, a main body including the internal space, and a cover which covers a part of the internal space. In this case, the visible region is not covered by the cover in the internal space, and includes an opened part adjacent to the cover.

It is preferable that the visible region should make the puncture needle visually recognizable.

The main body includes, for example, a wall which defines the internal space. It is preferable that the wall should include a recess which allows the puncture needle of the lancet to move, and configures a part of the visible region.

The lancet comprises, for example, a lancet main body including the puncture needle and an engaging part, and a cap which covers a leading end of the puncture needle and is detachable from the lancet main body. In this case, it is preferable that the casing should comprise a first stopper and a second stopper which restrain the engaging part, and substantially restrict a movement of the lancet in the internal space.

The casing may comprise a stopper for regulating an insertion depth to the puncture device when attached to the puncture device.

The lancet device of the present invention may further comprise an analytical instrument retained in the casing. In this case, it is preferable that the casing should comprise a flow path for supplying a fluid flowing out from the target site to the analytical instrument.

The flow path extends along, for example, a moving route of the puncture needle, and includes a suction part to be contacted with the target site. Moreover, the flow path may further include a through-hole opened in a retaining face on which the analytical instrument is held.

The present invention according to a second aspect provides a puncture device to which a lancet device is attached and which is for piercing a target site, wherein the puncture device employs the lancet device according to the first aspect of the present invention as the lancet device.

In a case in which the lancet device comprises an analytical instrument, the puncture device according to the second aspect may further comprise an analyzing mechanism which analyzes a specific constituent in the fluid using the analytical instrument.

The present invention according to a third aspect provides a puncture device for moving a puncture element in a piercing direction from a stand-by position toward a piercing position, and for piercing a target site with the puncture element, and the puncture device comprises a first member which is movable in the piercing direction and in an evacuating direction opposite to the piercing direction, a second member which is movable in the piercing direction and in the evacuating direction together with a motion of the first member, and a third member which is movable together with the puncture element and the second member, and is movable in the piercing direction and in the evacuating direction.

It is preferable that the puncture device according to the third aspect should further comprise a fourth member for interconnecting the first member and the second member together, and for converting a motion of the first member into a reciprocating motion of the second member.

The fourth member comprises, for example, a rotating shaft fixed at a certain position, a first movable part which engages with the first member, and is rotatable around the rotating shaft, and a second movable part which engages with the second member, and is rotatable around the rotating shaft.

The first member includes, for example, a first engaging part for allowing the first movable part to rotate, and on the other hand, the second member includes, for example, a second engaging part for allowing the second movable part to rotate.

The first engaging part includes, for example, an inclined part inclined relative to the piercing direction and the evacuating direction. In this case, it is preferable that the second member should be reciprocated in the piercing direction or in the evacuating direction as the second movable part moves through the second engaging part when the first movable part moves through the inclined part.

The fourth member is biased toward, for example, the evacuating direction, and is selectable a condition in which the fourth member is movable together with the first member, and a condition in which the fourth member is movable together with the second member.

The first engaging part may further include a straight line part connected to an end of the inclined part in the evacuating direction. In this case, the third member is movable together with the first member without moving the second member and the fourth member in the piercing direction and in the evacuating direction when, for example, the first movable part moves through the straight line part.

The puncture device according to the third aspect is to be used together with a lancet device comprising the puncture element and an analytical instrument attached thereto, and the puncture device may further comprise an analyzing mechanism which analyzes a specific constituent in the fluid using the analytical instrument.

### Brief Description of Drawings

FIG. 1 is an overall perspective view showing an analyzer and a sensor/lancet integrated device according to the present invention;
FIG. 2 is a front view showing an interior of the analyzer;
FIG. 3A and FIG. 3B are overall perspective views showing the sensor/lancet integrated device;
FIG. 4A to FIG. 4C are front views for explaining a casing main body in the sensor/lancet integrated device;
FIG. 5 is a cross-sectional view along a line V-V in FIG. 3A;
FIG. 6 is a cross-sectional view showing an essential part in FIG. 5 enlarged;
FIG. 7A is a front view for explaining a lancet in the sensor/lancet integrated device, and FIG. 7B is a cross-sectional view thereof;
FIG. 8 is an overall perspective view for explaining a biosensor in the sensor/lancet integrated device;
FIG. 9 is an exploded perspective view for explaining a piercing mechanism in the analyzer;
FIG. 10 is a side view for explaining a link member in the piercing mechanism with a portion thereof being shown in section;
FIG. 11 is a front view for explaining the link member in the piercing mechanism;
FIG. 12A to FIG. 12C are front views showing a relationship between a first moving member and the link member when a first movable pin of the link member moves through an inclined groove of the first moving member in the piercing mechanism in a direction N1;
FIG. 13A to FIG. 13C are front views showing a relationship between the first moving member and the link member when the first movable pin of the link member moves through the inclined groove of the first moving member in the piercing mechanism in a direction N2;
FIG. 14A to FIG. 14C are front views showing a relationship between the first moving member and the link member when the first movable pin of the link member moves through a straight line groove of the first moving member in the piercing mechanism;
FIG. 15A to FIG. 15D are front views showing a relationship between the link member and a second moving member when the link member in the piercing mechanism rotates;
FIG. 16A and FIG. 16B are perspective views showing a lancet holder in the piercing mechanism;
FIG. 17A to FIG. 17D are front views for explaining an operation of retaining the lancet in the sensor/lancet integrated device in the lancet holder in the piercing mechanism;
FIG. 18A and FIG. 18B are front views for explaining an operation when a cap is detached from the lancet with the lancet being retained in the lancet holder in the piercing mechanism;
FIG. 19A to FIG. 19C are front views for explaining an operation of piercing a skin with the piercing mechanism and the lancet; and
FIG. 20A to FIG. 20C are front views for explaining an operation of detaching a device from the lancet holder.

### Description of reference numerals

- 1: Analyzer (puncture device)
- 10: Piercing mechanism
- 11: Analyzing mechanism
- 2: Lancet device
- 3: Casing
- 30: Casing main body
- 31: Cover
- 32A: Stopper
- 32B: (first) Stopper
- 32C: (second) Stopper
- 33: Rear wall (second wall)
- 33C: Through-hole (flow path)
- 34: Lower wall (first wall)
- 34A: Recess
- 34B: Suction part (flow path)
- 35: Internal space
- 36: Opening (visible region)
- 4: Lancet (puncture element)
- 40: Lancet main body (main body)
- 41: Cap
- 43: Engaging part
- 44: Puncture needle
- 5: Biosensor (analytical instrument)
- 60: Link member (fourth member)
- 60A: First movable pin (of the link member) (first movable part)
- 60B: Second movable pin (of the link member) (second movable part)
- 60C: Fixed shaft (of the link member) (rotating shaft)
- 61: First moving member (first member)
- 61A: Groove (of the first moving member) (first engaging part)
- 61Aa: Inclined groove (inclined part)
- 61Ab: Straight line groove (straight line part)
- 62: Second moving member (second member)
- 62A: Groove (of the second moving member) (second engaging part)
- 63: Lancet holder (third member)
- N1: Piercing direction
- N2: Evacuating direction

### Best Mode for Carrying Out the Invention

An explanation will be given of the present invention in detail with reference to the accompanying drawings.

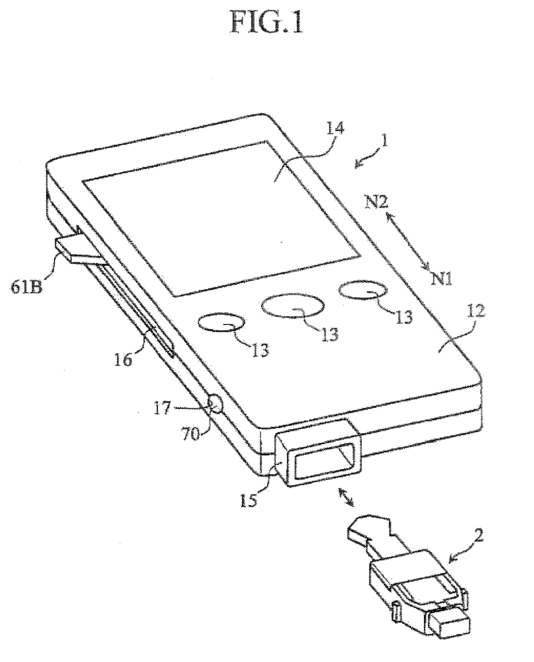

An analyzer 1 shown in FIG. 1 is to be used together with a sensor/lancet integrated device 2 (hereinafter, simply called a "device 2") attached thereto, and is configured as a handy type which facilitates carrying thereof. The analyzer 1 has both function of piercing a skin and function of analyzing a specific constituent in a blood using the blood caused to flow out through the skin.

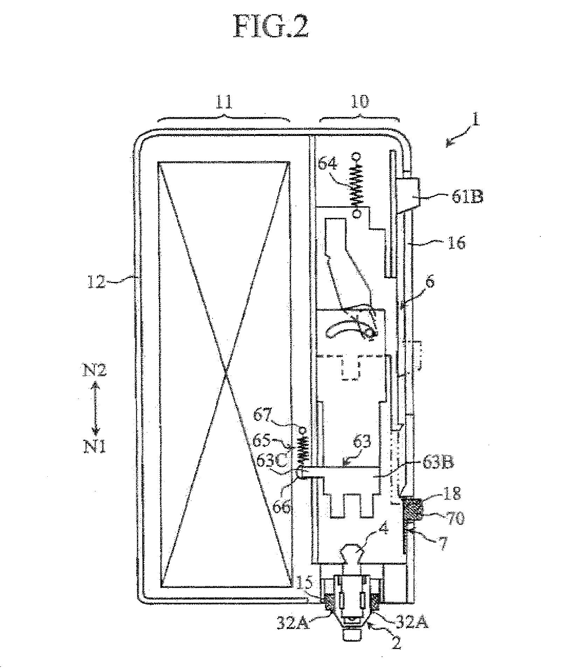

As shown in FIG. 2, the analyzer 1 employs a configuration that a piercing mechanism 10 for realizing the function of piercing a skin and an analyzing mechanism 11 which realizes the function of analyzing a specific constituent are retained in an interior of a housing 12.

As shown in FIG. 1, the housing 12 is for defining a contour of the device, and operation buttons 13 and a display panel 14 are provided on a surface of the housing 12. The operation button 13 is pressed for generating a signal for various operations (e.g., an analyzing operation or a printing operation), or is pressed for various settings (e.g., setting of an analysis condition, or inputting of an ID of a human subject). The display panel 14 is for displaying an analysis result or an error indication, and is also for displaying an operation procedure at the time of setting, and an operation condition.

As shown in FIG. 1 and FIG. 2, a device retaining part 15 is further provided on a side face of the housing 12, and a slit 16 and a through-hole 17 are provided in another side face of the housing 12. The device retaining part 15 is for retaining the device 2. The slit 16 is for allowing an operation part 61B of the piercing mechanism 10 to be discussed later to move. The through-hole 17 is for exposing a button 70 of the piercing mechanism to be discussed later.

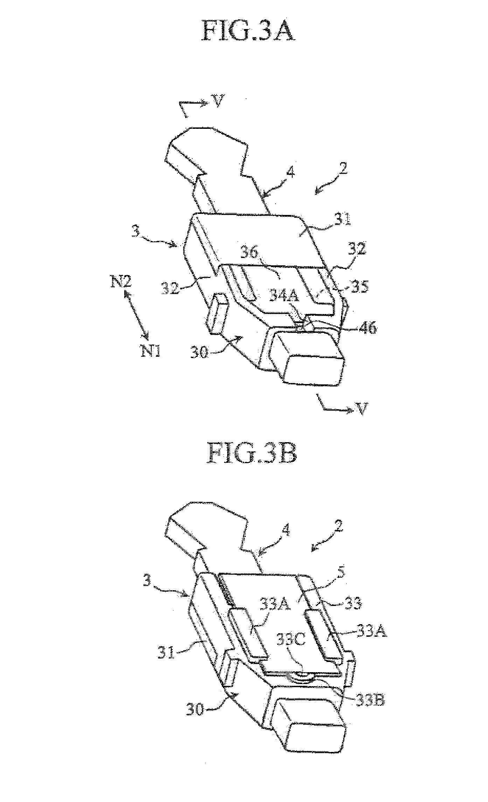

The device 2 is for enabling piercing and analysis of the analyzer 1, and as shown in FIG. 3A and FIG. 3B, comprises a casing 3, a lancet 4 and a biosensor 5.

The casing 3 is retained in the device retaining part 15 of the analyzer 1 (see FIG. 1 and FIG. 2), and is for retaining the lancet 4 and the biosensor 5 therein. The casing 3 comprises a casing main body 30 and a cover 31.

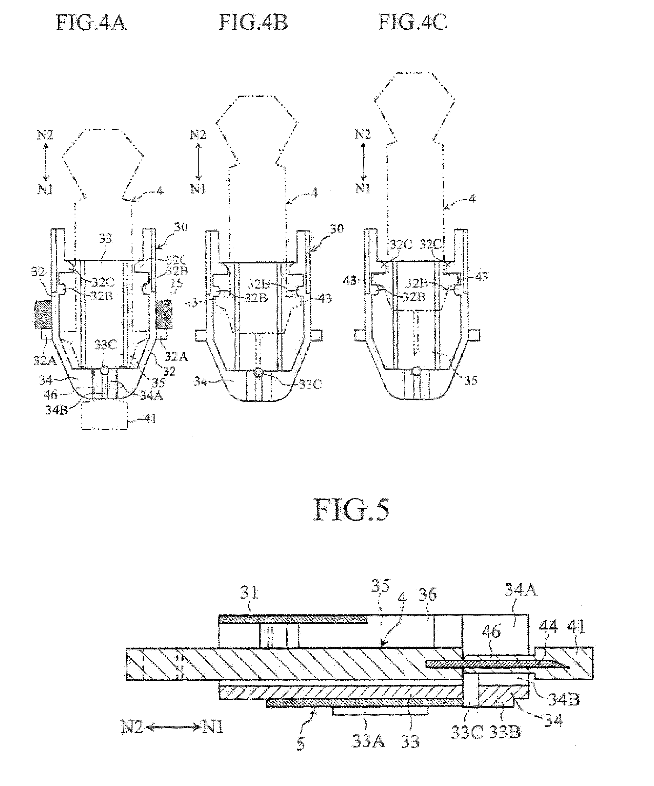

As shown in FIG. 4A, the casing main body 30 configures a major part of the casing 3, and has an internal space 35 defined by a pair of side walls 32, a rear wall 33 and a lower wall 34.

Each of the side walls 32 has stoppers 32A, 32B and 32C. The stopper 32A interferes with the device retaining part 15 when the device 2 is attached to the device retaining part 15 of the analyzer 1, and protrudes outwardly from the side wall 32. That is, the device 2 has an insertion depth in the device retaining part 15 regulated by the stopper 32A. As shown in FIG. 4B, the stopper 32B causes an engaging part 43 of the lancet 4 to be discussed later to interfere, and is for restraining the lancet 4 movable by a fixed distance in directions N1 and N2 together with the lower wall 34. That is, the lancet 4 is movable within a certain range in the directions N1 and N2 in the internal space 35. As shown in FIG. 4C, the stopper 32C is for retaining the engaging part 43 together with the stopper 32B, and is for restraining the lancet 4.

As shown in FIG. 3A and FIG. 5, the cover 31 is for interconnecting the pair of side walls 32 together. The cover 31 is for restraining the lancet 4 together with the rear wall 33 in a thickness direction in the internal space 35. The cover 31 has a smaller dimension in the directions N1 and N2 than that of the side wall 32, and selectively interconnects only upper parts of the side walls 32 together. Accordingly, the internal space 35 is partially covered, and a part of the internal space 35 is exposed, so that a leading end of the lancet 4 is visually recognizable. That is, an adjoining part of the cover 31 in the direction N1 is an opening 36 for exposing the lancet 4.

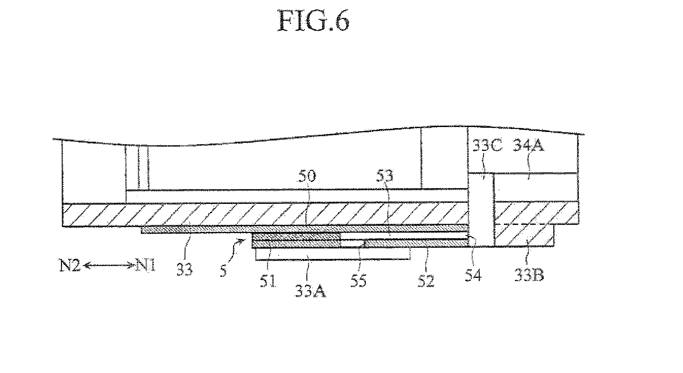

As shown in FIG. 3B. FIGS. 5 and 6, the rear wall 33 has a positioning parts 33A, 33B and a through-hole 33C. The positioning part 33A functions as a guide when the biosensor 5 is attached to the casing main body 30, and is for positioning and holding the biosensor 5 when the biosensor 5 is attached. The positioning 33B is for defining a position of the biosensor 5 together with the positioning part 33A, and is formed so as to protrude from the rear wall 33. The through-hole 33C is for making capillary force effective, and is for supplying a blood to the biosensor 5 together with a suction part 34B of the lower wall 34 to be discussed later. The through-hole 33C is provided so as to pass all the way through the rear wall 33, is communicated with the suction part 34B, and is opened in a surface of the rear wall 33. The through-hole 33C is communicated with the suction part 34B, and configures a flow path together with the suction part 34B. The through-hole 33C permits a blood sucked by the suction part 34B to be supplied therethrough, and to be supplied to the biosensor 5. The through-hole 33C can also supply the blood to the biosensor 5 as the blood is directly supplied without flowing through the suction part 34B.

As shown in FIG. 4A and FIG. 5, the lower wall 34 is for regulating a position of the lancet 4 and a moving range thereof in the directions N1 and N2, and has a recess 34A and the suction part 34B. The recess 34A is for retaining a narrow part 46 of a cap 41 of the lancet 4 to be discussed later. The lower wall 34 is also for allowing a puncture needle 44 in the lancet 4 to move with the cap 41 being detached from the lancet 4. That is, the recess 34A also has a function of making a puncture 41 in a lancet 41 visually recognizable, and configures a part of a visible region. The suction part 34B is for suctioning a blood caused to flow out through a skin by capillary force, and is formed in a groove-like shape opened laterally. The suction part 34B is communicated with the through-hole 33C, and allows the blood sucked by the suction part 34B to be supplied to the through-hole 33C.

The casing 3 employs a very simple configuration including the casing main body 30 and the cover 31, and can be easily formed by, for example, resin molding. Accordingly, it becomes possible to suppress any effort of processing the casing 3 and increasing of a cost thereof.

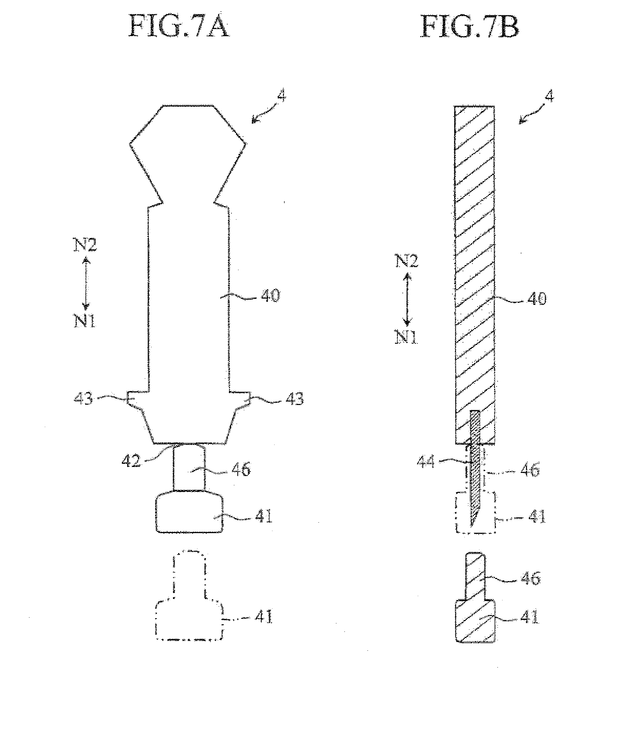

As shown in FIG. 7A and FIG. 7B, the lancet 4 is retained in a lancet holder 63 (see FIG. 2) in the piercing mechanism 10 to be discussed later, and is used for piercing a skin. The lancet 4 has a lancet main body 40, the cap 41 and a brittle part 42,

The lancet main body 40 is retained in the lancet holder 63 (see FIG. 2), and has the engaging part 43 and the puncture needle 44. The engaging part 43 is engaged with the stopper 32B of the casing 3, and is for preventing the lancet 4 from carelessly ejecting out from the casing 3 in the direction N2 (see FIG. 4B). The puncture needle 44 is fixed in the lancet main body 40, and has a needle tip 45 protruding from the lancet main body 40. The cap 41 is for covering the needle tip 45 of the puncture needle 44, and is detachable from the lancet main body 40. When the cap 41 is detached from the lancet 4, as the needle tip 45 of the puncture needle 44 protrudes from the lancet main body 40, the needle tip of the puncture needle 44 is in an exposed condition. The cap 41 has the narrow part 46. As shown in FIG. 3A, FIG. 4A and FIG. 5, the narrow part 46 is retained in the recess 34A of the lower wall 34 of the casing main body 30. With the narrow part 46 being retained in the recess 34A, the position of the lancet 4 relative to the casing main body 30 (the casing 3) is regulated, and the lancet 4 is restrained in the directions N1 and N2. The brittle part 42 is for facilitating detachment of the cap 41 from the lancet main body 40.

The lancet 4 can be formed by inserting the puncture needle 44 therein by resin molding. Moreover, as a sterilizing process is performed on the lancet after the lancet is formed, the lancet 4 can maintain a clean condition until the cap 41 is detached and the needle tip 45 of the puncture needle 44 is exposed. Furthermore, by devising the shape of the casing main body 30 of the casing 3, the existing lancet 4 can be also used. Accordingly, it becomes possible to reduce the production cost of the lancet 4, thereby reducing the production cost of the device 2.

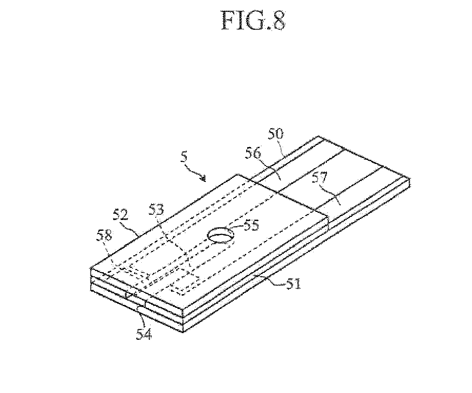

As shown in FIG. 8, the biosensor 5 employs a configuration that a cover 52 is stacked on a long-rectangular substrate 50 via a spacer 51. A capillary 53 extending in a lengthwise direction of the substrate 50 is defined by each of structural elements 50 to 52 in the biosensor 5.

The capillary 53 is for moving a blood introduced from an end 54 to a through-hole 55 of the cover 52 using an capillary phenomenon, and is for retaining the introduced blood.

Formed on an upper face of the substrate 50 are an acting electrode 56, a counter electrode 57 and a reagent part 58. The acting electrode 56 and the counter electrode 57 are caused to contact connectors (not illustrated) of the analyzer 1 when the device 2 is attached to the analyzer 1. The acting electrode 56 and the counter electrode 57 enable application of a voltage to a blood in the capillary 53, and enable measurement of a response current when the voltage is applied.

The reagent part 58 is arranged in the interior of the capillary 53, and contains, for example, an electron transferring substance and an oxidoreductase. Examples of the oxidoreductase are a glucose oxidase (GOD) and a glucose dehydrogenase (GDH) when a glucose in a blood is analyzed, and a PQQGDH is typically used. Examples of the electron transferring substance are a ruthenium complex and an iron complex, and an [Ru(NH₃)₆]CI₃ or a K₃[Fe(CN)₆] can be typically used. When a constituent, such as a lactic acid or a cholesterol, other than the glucose in the blood is analyzed, it is needless to say that an oxidoreductase and an electron transferring substance in accordance with the analysis target are used.

As the biosensor 5, an existing biosensor can be also used by devising the respective positions of the positioning parts 33A and 33B of the casing main body 30 of the casing 3 and the respective shapes thereof. This enables reduction of the production cost of the biosensor 5, thereby reducing the production cost of the device 2.

As shown in FIG. 2, with a blood being supplied to the biosensor 5, the analyzing mechanism 11 is for analyzing a specific constituent in the blood from a response current when the voltage is applied across the acting electrode 56 and the counter electrode 57 (see FIG. 6 and FIG. 8). The analyzing mechanism 11 has information indicating, for example, a relationship between the response current and a concentration of the specific constituent (e.g., a standard curve or a corresponding table), and enables analysis of the concentration or the like of the specific constituent in the blood by comparing the information with a measured response current.

As shown in FIG. 2, the piercing mechanism 10 is used for moving the lancet 4 in the device 2 from a stand-by position to a piercing position to pierce a skin, and is for causing a blood to flow out through the skin. The piercing mechanism 10 has a lancet moving mechanism 6 and a latch releasing member 7.

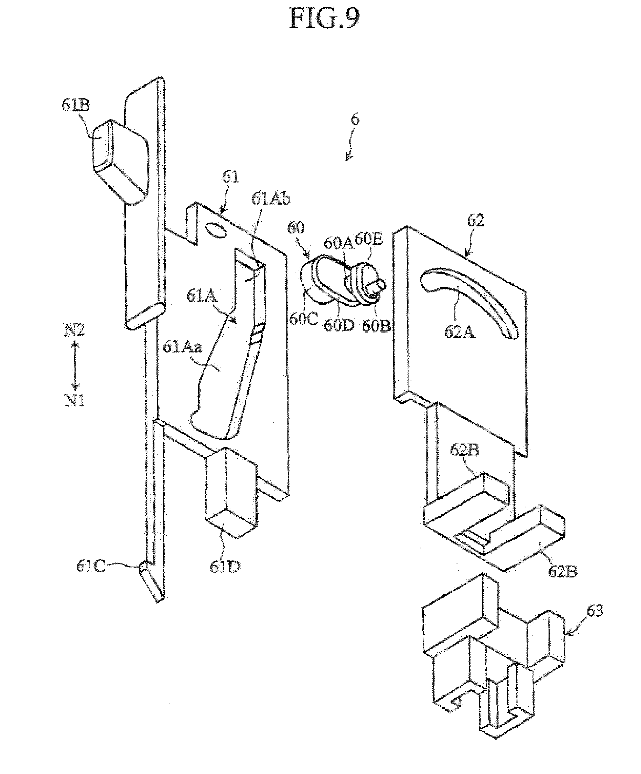

As shown in FIG. 9, the lancet moving mechanism 6 has a link member 60, a first moving member 61, a second moving member 62 and the lancet holder 63. The lancet moving mechanism 6 converts the reciprocating motion of the first moving member 61 into the reciprocating motion of the lancet holder 63 through the circular motion of the link member 60.

As shown in FIG. 9 to FIG. 11, the link member 60 is for interlocking the second moving member 62 with the movement of the first moving member 61 when the first moving member 61 moves and for moving the second moving member 62. The link member 60 has a first movable pin 60A, a second movable pin 60B, a fixed shaft 60C, a first arm 60D and a second arm 60E.

The first movable pin 60A engages with the first moving member 61, and is for connecting the first arm 60D and the second arm 60E together. More specifically, the first movable pin 60A fixes an end 60Db of the first arm 60D and an end 60Eb of the second arm 60E together in a shifted condition from each other at an end 60Da of the first arm 60D and an end 60Ea of the second arm 60E.

The second movable pin 60B engages with the second moving member 62, and protrudes in a direction opposite to the first movable pin 60A at the end 60Eb of the second arm 60E.

The fixed shaft 60C is for fixing the link member 60 rotatable relative to the housing 12, and protrudes in a direction opposite to the first movable pin 60A at the end 60Db of the first arm 60D.

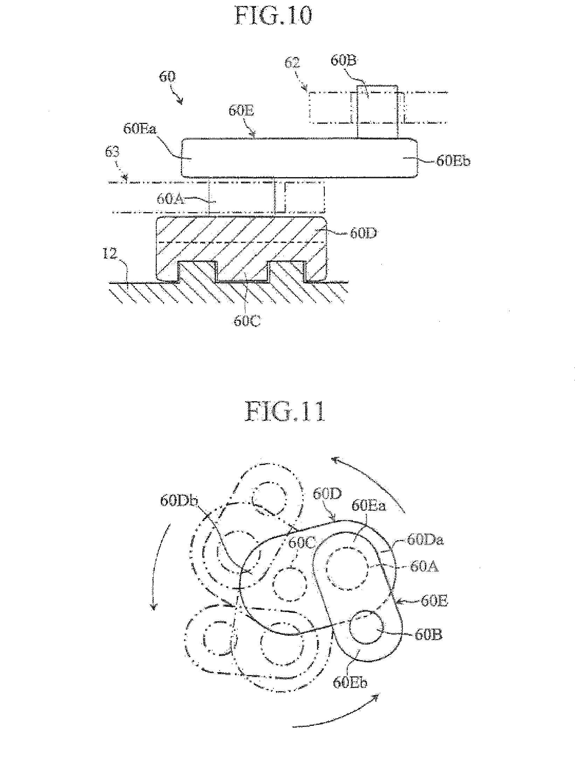

In the link member 60, as shown in FIG. 11, the first movable pin 60A, the second movable pin 60B and the fixed shaft 60C are connected together via the first arm 60D and the second arm 60E, so that positional relationships among the first movable pin 60A, the second movable pin 60B and the fixed shaft 60C are regulated. More specifically, the first movable pin 60A and the second movable pin 60B have the same distance to the fixed shaft 60C, and are rotatable over a circumference around the fixed shaft 60C with respective phases being shifted.

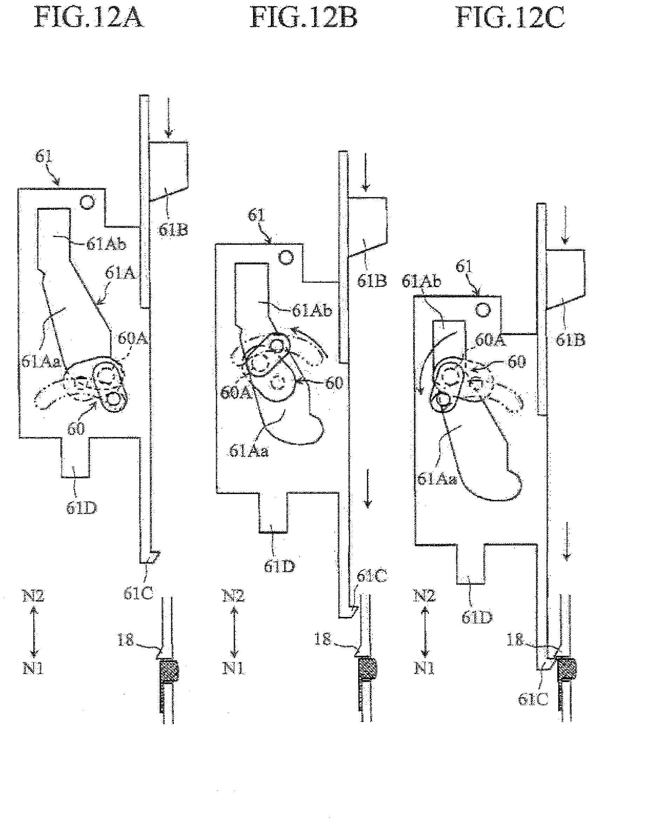

As shown in FIG. 9, and FIG. 12A to FIG. 12C, the first moving member 61 is movable relative to the housing 12 in the directions N1 and N2, and as shown in FIG. 2, is connected to the housing 12 via a coil spring 64 (see FIG. 2). The first moving member 61 has a groove 61A, the operation part 6 1 B, a hook 61C and a push-down part 61D.

The groove 61A is for allowing the first movable pin 60A of the link member 60 to move. The groove 61A has an inclined groove 61Aa extending in a direction inclined to the directions N1 and N2, and a straight line groove 61Ab connected to an end of the inclined groove 61 Aa.

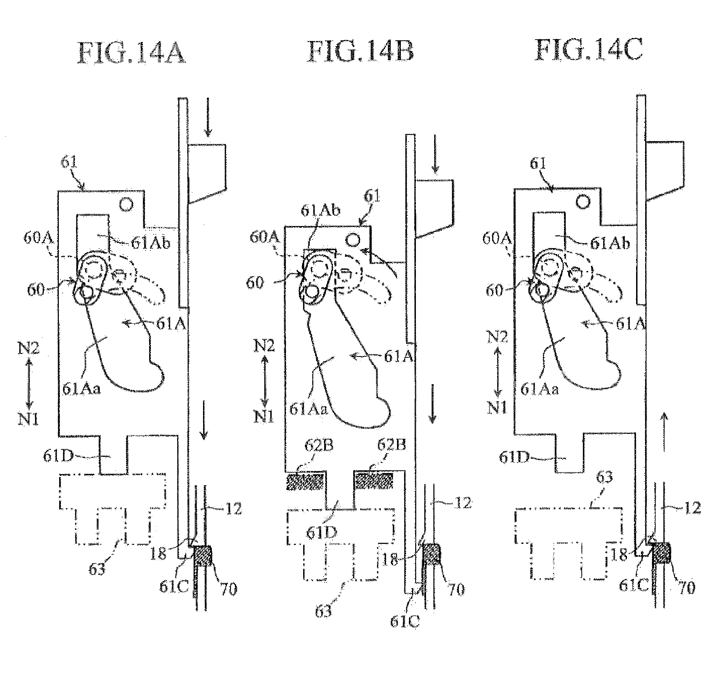

As is clear from FIG. 12A to FIG. 13C, the inclined groove 61Aa is for rotating the first movable pin 60A when the first moving member 61 is moved in the directions N1 and N2. As shown in FIG. 14A to FIG. 14C, the straight line groove 61Ab is for allowing the first moving member 61 to move in the directions N1 and N2 without rotating the first movable pin 60A.

As shown in FIG. 12A to FIG. 14C, the operation part 61B is used when the first moving member 61 is manually moved. The operation part 61B has a part protruding outwardly through the slit 16 of the housing 12, and is allowed by the slit 16 to move in the directions N1 and N2 (see FIG. 1 and FIG. 2).

As shown in FIG. 14C, the hook 61C is for latching the first moving member 61 with the housing 12 by engaging with a protruding part 18 of the housing 12.

As shown in FIG. 14A to FIG. 14C, the push-down part 61D moves the lancet holder 63 in the direction N1 when the first moving member 61 is moved in the direction N1 below a position (a latching position) where the hook 61C is latched with the protruding part 18 of the housing 12. The push-down part 61D protrudes in the direction N1 at a lower part of the first moving member 61, and is movable between a pair of flanges 62B of the second moving member 62 to be discussed later.

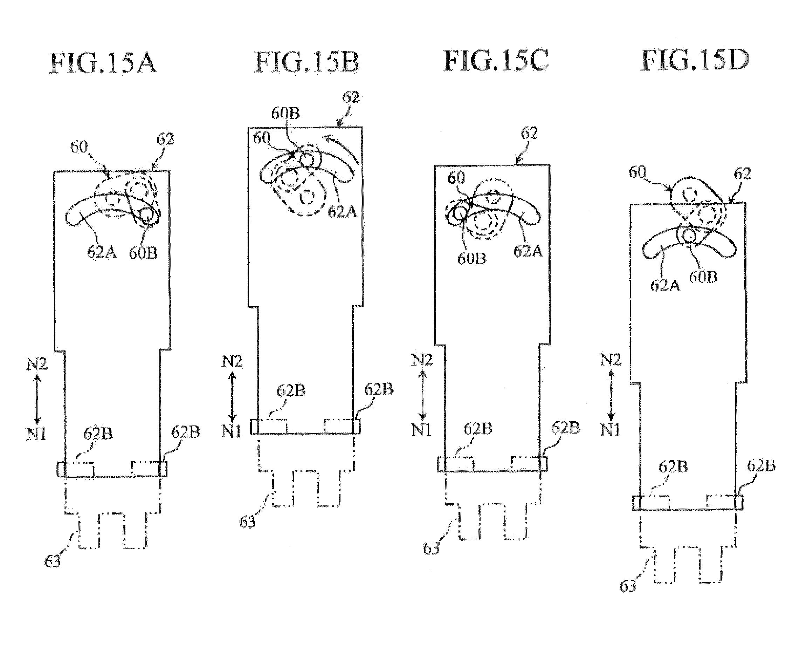

As shown in FIG. 9, and FIG. 15A to FIG. 15C, the second moving member 62 is for reciprocating the lancet holder 63 in the directions N1 and N2 when the hook 61C of the first moving member 61 is located above the latching position. The second moving member 62 has a groove 62A and the pair of flanges 62B.

The groove 62A engages with the second movable pin 60B while allowing the rotational motion of the second movable pin 60B. The groove 62A is formed in an arc shape, and as the second movable pin 60B engages with the groove 62A with a rotational motion, the second moving member 62 is moved in the directions N1 and N2. That is, when the first moving member 61 is moved in the directions N1 and N2, the second moving member 62 is moved together with the rotational motion of the link member 60 (the first movable pin 60A) in the directions N1 and N2.

The pair of flanges 62B are for moving the lancet holder 63 in the directions N1 and N2 when the second moving member 62 moves in the directions N1 and N2. The flanges 62B are provided so as to interfere with the lancet holder 63 when the hook 61C of the first moving member 61 moves in the directions N1 and N2 above the latching position (see FIG. 12A to FIG. 12C). The pair of flanges 62B are spaced apart from each other, and when the first moving member 61 moves below the latching position in a clearance 62C (see FIG. 14A to FIG. 14C), the push-down part 61D of the first moving member 61 is allowed to move.

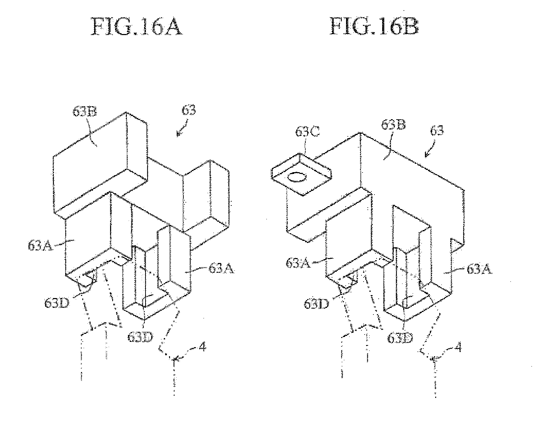

As shown in FIG. 2, FIG. 16A and FIG. 16B, the lancet holder 63 is for retaining the lancet 4 of the device 2, and is for moving the lancet 4. The lancet holder 63 has a pair of engaging parts 63A, a block 63B and a latching part 63C.

The pair of engaging parts 63A are for retaining the lancet 4, and grooves 63D are formed respectively therein. The grooves 63D are for fitting an end of the lancet 4 therein. The grooves 63D in the pair of engaging parts 63A are formed at positions facing each other.

The block 63B is for interfering with the push-down part 61D of the first moving member 61 or the flange 62B of the second moving member 62 (see FIG. 14A to FIG. 14C, or (see FIG. 15A to FIG. 15D)).

As shown in FIG. 2, the latching part 63C is for latching with an end 66 of a coil spring 65, and is protrudingly provided from the block 63B. The coil spring 65 has an end 67 fixed to the housing 12, and force directed toward the direction N2 acts on the block 63B (the lancet holder 63). That is, the block 63B (the lancet holder 63) is permitted by the coil spring 65 to maintain a condition in which the block contacts the push-down part 61D of the first moving member 61 or the flange 62B of the second moving member 62 (see, FIG. 14A to FIG. 14C, or (see FIG. 15A to FIG. 15D)).

As shown in FIG. 15A to FIG. 15D, the lancet holder 63 can be moved in the directions N1 and N2 by the second moving member 62 when the hook 61C of the first moving member 61 is located above the latching position, and as shown in FIG. 14A to FIG. 14C, the lancet holder can be moved in the direction N1 by the first moving member 61 when the hook 61C of the first moving member 61 is located below the latching position.

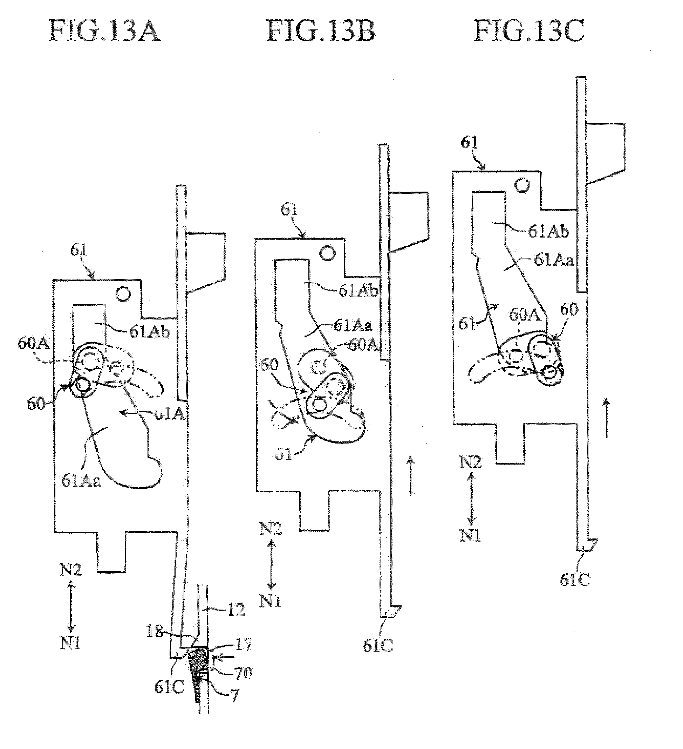

As shown in FIG. 13A, the latch releasing member 7 is for releasing a latching condition of the first moving member 61 relative to the housing 12 (the protruding part 18), and has the button 70 and a plate spring 71. The button 70 is exposed from the side face of the housing 12, and can wobble. The plate spring 71 has moderate elasticity, extends from the button 70 and is fixed to the housing 12 at an end 72.

In the above-explained latch releasing member 7, when the button 70 is pressed, the button 70 acts on the hook 61C of the first moving member 61, thereby releasing engagement of the hook 61C with the protruding part 18 of the housing 12.

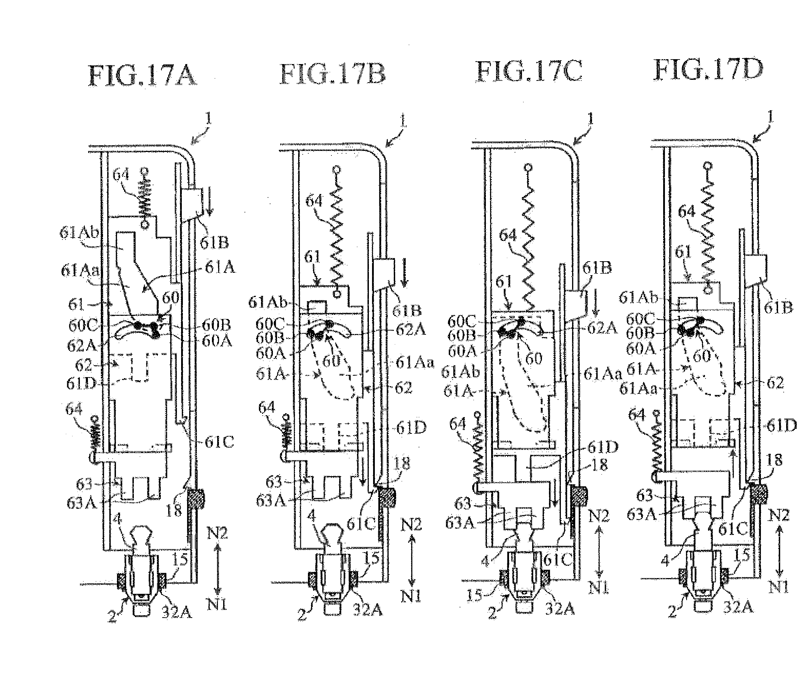

It will be explained below how to use the analyzer 1 and an operating principle thereof. However, in an initial condition, the first moving member 61, the second moving member 62, the link member 60 and the lancet holder 63 have respective positional relationships as shown in FIG. 17A.

When a skin is pierced using the analyzer 1, first, the device 2 is retained in the device retaining part 15 of the housing 12. Such an operation is performed by inserting the device 2 into the device retaining part 15. At this time, as the casing 3 of the device 2 is provided with the stopper 32A, the insertion depth (position) of the device 2 is regulated as the stopper 32A interferes with the device retaining part 15 (see FIG. 2 and FIG. 4A). As the device 2 is larger than the lancet 4 and the biosensor 5, the attachment of the device 2 to the analyzer 1 can be performed easily in comparison with a case in which the lancet 4 and the biosensor 5 are individually attached to the analyzer 1

Next, as shown in FIG. 17A to FIG. 17D, the lancet 4 of the device 2 is retained in the lancet holder 63, and the hook 61C of the first moving member 61 is engaged with the protruding part 18 of the housing 12. Such an operation is accomplished by pushing down the operation part 61B of the first moving member 61 in the direction N1 by a predetermined distance. As shown in FIG. 17A and FIG. 17B, when the first moving member 61 is moved in the direction N1, the hook 61C reaches a position engageable with the protruding part 18 of the housing 12. At this time, the coil spring 64 is stretched, and the first movable pin 60A of the link member 60 moves through the inclined groove 61 Aa and the link member 60 rotates around the fixed shaft 60C in a counterclockwise direction in the figures. Together with this operation, the second movable pin 60B of the link member 60 rotates in the counterclockwise direction in the figures, and the second moving member 62 moves upwardly in the direction N2, and then returns to the original position (see FIG. 15A to FIG. 15C).

As shown in FIG. 17A and FIG. 17B, when the operation part 61B is further pushed down in the direction N1, the hook 61C moves downwardly of the protruding part 18. At this time, the first movable pin 60A of the link member 60 moves through the straight line groove 61Ab, and the first moving member 61 moves in the direction N1 relative to the second moving member 62 without rotating the link member 60. As a result, the push-down part 61D of the first moving member 61 interferes with the lancet holder 63, and the lancet holder 63 is moved by the first moving member 61 in the direction N1. Accordingly, the lancet 4 fits into the groove 63D of the engaging part 63A of the lancet holder 63 (see FIG. 16A and FIG. 16B), and the coil springs 64 and 65 are stretched.

As shown in FIG. 17C and FIG. 17D, when a load acting on the operation part 61B is released, as the coil spring 64 is stretched, the first moving member 61 is moved in the direction N2 by the elastic force of the coil spring 64. As a result, the hook 61C is latched with the protruding part 18. At this time, as the first movable pin 60A moves through the straight line groove 61Ab of the first moving member 61, the link member 60 does not rotate, and the second moving member 62 does not move, too.
With the hook 61C being latched with the protruding part 18, as the first moving member 61 is located below an initial position (see FIG. 17A), the coil spring 65 is sufficiently stretched.

On the other hand, the lancet does not move because the narrow part 46 of the cap 41 is engaging with the recess 34A of the casing main body 30 (see, FIG. 4A). At this time, because the lancet 4 is fitted into the lancet holder 63, the lancet holder 63 also does not move, and the lancet holder 63 is detached from the first moving member 61.

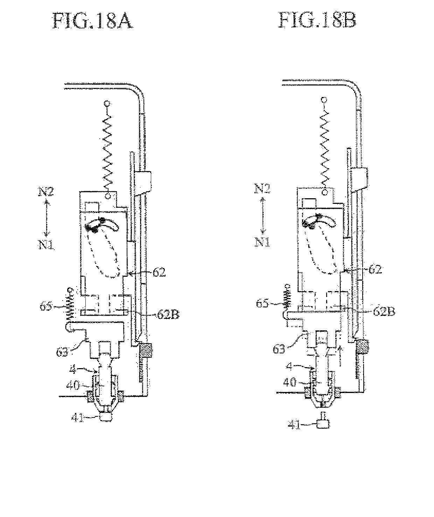

As shown in FIG. 18A and FIG. 18B, with the lancet 4 being retained in the lancet holder 63, when the cap 41 is detached from the lancet 4, the puncture needle 44 is exposed from the lancet main body 40 (see FIG. 7B). Meanwhile, with regard to the lancet main body 40, as the restraint of the device 4 in the recess 34A of the rear wall 34 in the casing main body 30 is released (see FIG. 4B), the lancet 4 moves together with the lancet holder 63 in the direction N2 by the elastic force of the coil spring 65. The movement of the lancet 4 in the direction N2 is stopped as the lancet holder 63 interferes with the flange 62B of the second moving member 62.

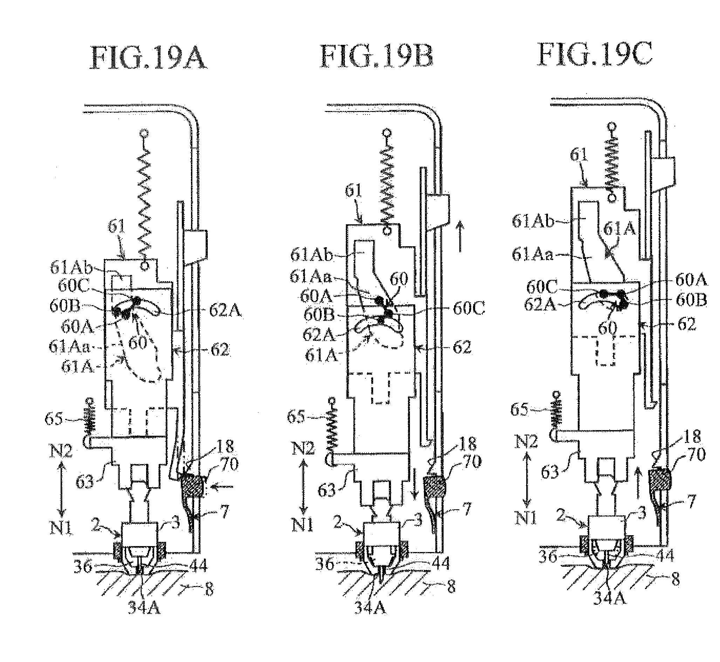

When latching of the first moving member 61 and attachment of the lancet 4 complete, as shown in FIG. 19A to FIG. 19C, with an end of the device 2 (the casing 3) being pressed against a skin 8, the skin is pierced as the button 70 of the latch releasing member 7 is pressed. As shown in FIG. 19A, when the button 70 is pressed, as the button 70 is displaced inwardly, the button 70 moves the hook 61C inwardly. Accordingly, latching of the hook 61C with the protruding part 18 is released.

Meanwhile, as shown in FIG. 19B and FIG. 19C, because the first moving member 61 is connected to the stretched coil spring 64, the first moving member moves in the direction N2 by the elastic force of the coil spring 64. At this time, as the first movable pin 60A of the link member 60 moves through the first moving member 61 the inclined groove 61Aa, the link member 60 rotates around the fixed shaft 60C. On the other hand, as the link member 60 rotates, the second movable pin 60B moves through the groove 62A of the second moving member 62. As a result, the second moving member 62 moves in the direction N1, and then moves in the direction N2 together with the rotational trajectory of the second movable pin. At this time, as the lancet holder 63 is connected to the coil spring 65 and interferes with the flange 62B of the second moving member 62, the lancet holder moves together with the second moving member 62. As the lancet 4 is retained in the lancet holder 63, the lancet 4 also moves together with the second moving member 62 in the direction N1, and then moves in the direction N2.

As the puncture needle 44 is exposed from the lancet 4, when the lancet 4 is moved in the direction N1, the puncture needle 44 protrudes from the casing 3, and pierces the skin 8. Conversely, when the lancet 4 moves in the direction N2, the puncture needle 44 is pulled out from the skin 8, and the piercing operation completes.
In this manner, piercing of the skin 8 by the lancet 4 and pulling out of the puncture needle are performed together with the movement of the first moving member 61 in the direction N2. Accordingly, it is possible not only to reduce a pain as a time while the puncture needle 44 is piercing the skin 8 is reduced, but also to accomplish a safeness as the puncture needle 44 is retained in the device 2 (the casing 3) after piercing of the skin. Moreover, when the skin is pierced, the movement of the puncture needle 44 can be checked through the visible regions, such as the recess 34A of the device 2 and the opening 36 thereof (see FIG. 3A and FIG. 5). Accordingly, a user is released from an anxiety originating from the invisibility of the puncture needle 44 when the skin is pierced.

When the skin 8 is pierced, a blood flows out through the skin 8. As is clear with reference to FIG. 4 to FIG. 6, the blood is suctioned by the suction part 34B of the lower wall 34 in the casing main body 30. As explained above, in the casing 3, a lower part of the lancet 4 is exposed. Accordingly, the user can check the piercing operation of the lancet 4 (the puncture needle 44) to the skin 8 through the casing 3. Consequently, the user can check a flow-out amount of the blood when extracting the blood. Accordingly, the user can check whether or not the flow-out amount is a sufficient amount necessary for the analysis of the blood. As a result, when a flow-out amount is little, it becomes possible for the user to avoid false measurement inherent to lack of the blood by detecting the blood insufficiency at an early point and prompting the flow-out of the blood by, for example, massaging the skin.

The blood suctioned by the suction part 34B moves through the through-hole 33C by capillary force, and reaches an end of the capillary 53 (an edge of the through-hole 55 of the cover 52) of the biosensor 5. In the capillary 53, the blood is moved and retained by the capillary force. In the device 2, the blood flowing out through the skin is supplied to the biosensor 5 through the suction part 34B and the through-hole 33C. Accordingly, it is not necessary to position the end 54 of the capillary 53 of the biosensor 5 to a blood flow-out site of the skin 8 and to introduce the blood into the capillary 53 in order to supply the blood to the biosensor 5. Consequently, when the device 2 is used, an effort of introducing the blood into the biosensor 5 can be omitted.

Meanwhile, in the analyzer 1, a specific constituent in the blood is analyzed in the analyzing mechanism 11 (see FIG. 2). The analysis of the specific constituent is performed by comparing a response current after a certain time when a voltage is applied across the acting electrode of the biosensor 5 and the counter electrode thereof (see FIG. 6 and FIG. 8) with information indicating a relationship between a response current and the concentration of the specific constituent. An analysis result at this time is displayed on the display panel 4.

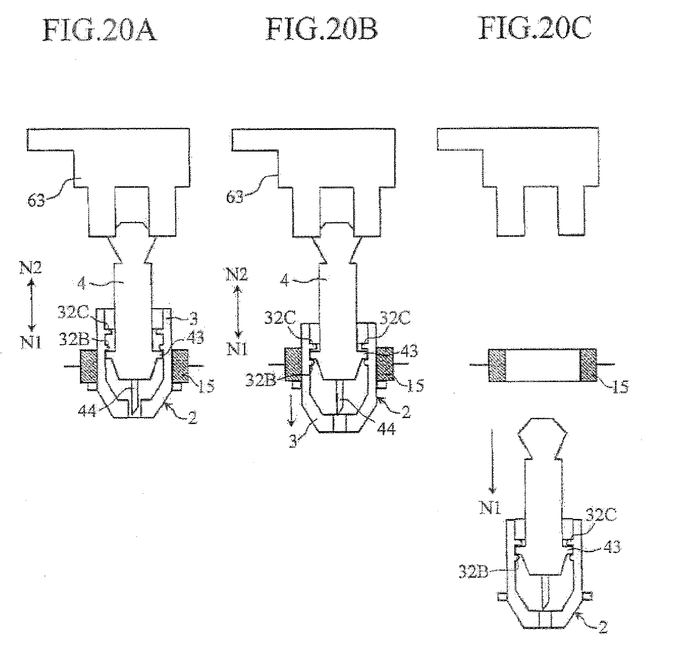

On the other hand, when the supply of the blood to the biosensor 5 completes, as shown in FIG. 20A to FIG. 20C, the device 2 is detached from the device retaining part 15 of the housing 12. The device 2 is detached as the device 2 is moved in the direction N1 relative to the housing 12.

As shown in FIG. 20A and FIG. 20B, when the device 2 is moved in the direction N1 relative to the housing 12, as the lancet 4 is retained in the lancet holder 63, the casing 3 moves in the direction N1 relative to the lancet 4. Accordingly, the engaging part 43 of the lancet 4 goes over the stopper 32B, and is restrained between the stopper 32B and the stopper 32C.

As shown in FIG. 20B and FIG. 20C, when the casing 3 is further moved in the direction N1, as the engaging part 43 of the lancet 4 is restrained between the stopper 32B and the stopper 32C, the lancet 4 is pulled out from the lancet holder 63. Accordingly, the device 2 is detached from the analyzer 1.

In the device 2 pulled out from the analyzer 1, the engaging part 43 of the lancet 4 keeps being restrained between the stopper 32B and the stopper 32C. Accordingly, in the device 2 after the use, the puncture needle 44 of the lancet 4 is prevented from protruding from the casing 3. As a result, the device 2 can be detached and discarded safely and hygienically.

As the device 2 is larger than the lancet 4 and the biosensor 5, the detachment of the device 2 from the analyzer 1 can be performed easily in comparison with a case in which the lancet 4 and the biosensor 5 are individually detached from the analyzer 1.

According to the above-explained analyzer 1, the first moving member 61 can be easily latched with the protruding part 18 of the housing 12 by pushing down the operation part 61B of the first moving member 61. Meanwhile, in order to pierce a skin, it is fine if the button 70 of the latch releasing member 7 is just pressed. In this manner, the analyzer 1 is convenient for use as the skin can be pierced through very simple operations, such as the downward movement of the operation part 61B and pressing of the button 70. Moreover, when an amount of a blood supplied to the biosensor 5 is insufficient, it is possible to pierce the skin again by downwardly moving the operation part 61B and pressing the button 70 again. Accordingly, it becomes possible to reduce the possibility of occurrence of any measurement error due to lack of a blood, or of any false measurement, and to reduce the number of the devices 2 to be discarded by efficiently using the device 2.

The analyzer of the present invention and the device 2 thereof are not limited to the above-explained embodiment, and can be changed and modified in various forms. For example, the piercing mechanism in the analyzer 1 is not limited to the mechanism employing the link member. Moreover, the biosensor 5 of the device 2 is not limited to an electrode type, and may be a colorimetric type, and may be affixed to a holder via a spacer with a cover being omitted.

The present invention is not limited to the analyzer, and is further applicable to a puncture device having no analytical function. In this case, the biosensor in the device is omitted.

## Claims

1. A lancet device which is attached to a puncture device and is used for piercing a target site, and which retains a lancet comprising a puncture needle in an internal space of a casing, wherein
the casing includes a visible region through which a leading end of the lancet is visually recognizable.

2. The lancet device according to claim 1, wherein
the casing comprises a main body which includes the internal space, and a cover which covers a part of the internal space, and
the visible region is not covered by the cover in the internal space, and includes an opened part adjacent to the cover.

3. The lancet device according to claim 1, wherein the visible region makes the puncture needle visually recognizable.

4. The lancet device according to claim 2, wherein
the main body includes a wall which defines the internal space, and
the wall includes a recess which allows the puncture needle of the lancet to move, and configures a part of the visible region.

5. The lancet device according to claim 1, wherein
the lancet comprises a lancet main body including the puncture needle and an engaging part, and a cap which covers a leading end of the puncture needle and is detachable from the lancet main body, and
the casing comprises a first stopper and a second stopper which restrain the engaging part, and substantially restrict a movement of the lancet in the internal space.

6. The lancet device according to claim 1, wherein the casing comprises a stopper for regulating an insertion depth to the puncture device when attached to the puncture device.

7. The lancet device according to claim 1, further comprising an analytical instrument retained in the casing, wherein
the casing comprises a flow path for supplying a fluid flowing out from the target site to the analytical instrument.

8. The lancet device according to claim 7, wherein the flow path extends along a moving route of the puncture needle, and includes a suction part to be contacted with the target site.

9. The lancet device according to claim 8, wherein the flow path further includes a through-hole opened in a retaining face on which the analytical instrument is held.

10. A puncture device to which a lancet device is attached and which is for piercing a target site, employing the lancet device according to claim 1 as the lancet device.

11. The puncture device according to claim 10, wherein the lancet device is the lancet device according to claim 7, and the puncture device further comprises:
an analyzing mechanism which analyzes a specific constituent in the fluid using the analytical instrument.

12. A puncture device for moving a puncture element in a piercing direction from a stand-by position toward a piercing position, and for piercing a target site with the puncture element, the puncture device comprising:
a first member which is movable in the piercing direction and in an evacuating direction opposite to the piercing direction;
a second member which is movable in the piercing direction and in the evacuating direction together with a motion of the first member; and
a third member which is movable together with the puncture element and the second member, and is movable in the piercing direction and in the evacuating direction.

13. The puncture device according to claim 12, further comprises a fourth member for interconnecting the first member and the second member together, and for converting a motion of the first member into a reciprocating motion of the second member.

14. The puncture device according to claim 13, wherein the fourth member comprises a rotating shaft fixed at a certain position, a first movable part which engages with the first member, and is rotatable around the rotating shaft, and a second movable part which engages with the second member, and is rotatable around the rotating shaft.

15. The puncture device according to claim 14, wherein
the first member includes a first engaging part for allowing the first movable part to rotate, and
the second member includes a second engaging part for allowing the second movable part to rotate.

16. The puncture device according to claim 15, wherein
the first engaging part includes an inclined part inclined relative to the piercing direction and the evacuating direction, and
the second member is reciprocated in the piercing direction or in the evacuating direction as the second movable part moves through the second engaging part when the first movable part moves along the inclined part.

17. The puncture device according to claim 12, wherein the fourth member is biased toward the evacuating direction, and is selectable a condition in which the fourth member is movable together with the first member, and a condition in which the fourth member is movable together with the second member.

18. The puncture device according to claim 17, wherein
the first engaging part further includes a straight line part connected to an end of the inclined part in the evacuating direction, and
the third member is movable together with the first member without moving the second member and the fourth member in the piercing direction and in the evacuating direction when the first movable part moves through the straight line part.

19. The puncture device according to claim 12, wherein the puncture device is to be used together with a lancet device, the lancet device comprising the puncture element and an analytical instrument and being attached thereto, and the puncture device further comprises an analyzing mechanism which analyzes a specific constituent in the fluid using the analytical instrument.
